# EUROPÄISCHE PATENTSCHRIFT

(11) **EP 1 015 037 B1**
(45) Veröffentlichungstag und Bekanntmachung des Hinweises auf die Patenterteilung: **29.06.2005**
(21) Anmeldenummer: 98948780.6
(22) Anmeldetag: 07.08.1998
(51) Int. Cl.: A61K 48/00

(54) **BEEINFLUSSUNG DER BINDUNG VON p53 AN ZIELGENE**
METHOD FOR INFLUENCING THE p53 LINKAGE IN TARGET GENES
PROCEDE POUR INFLUER SUR LA LIAISON DE p53 SUR DES GENES CIBLES

(30) Priorität: 14.08.1997 DE 19735221
(43) Veröffentlichungstag der Anmeldung: 05.07.2000
(73) Patentinhaber: Deppert, Wolfgang Willi, 22359 Hamburg (DE)
(72) Erfinder: DEPPERT, Wolfgang, Willi, D-22359 Hamburg (DE); KIM, Ella, D-22359 Hamburg (DE)
(74) Vertreter: Schüssler, Andrea, Dr.
(86) Internationale Anmeldenummer: PCT/DE1998/002326
(87) Internationale Veröffentlichungsnummer: WO 1999/008712

(56) Entgegenhaltungen:
- WO-A-97/11367
- WO-A-97/14794
- HANSEN S ET AL: "Allosteric regulation of the thermostability and DNA binding activity of human p53 by specific interacting proteins. CRC Cell Transformation Group." JOURNAL OF BIOLOGICAL CHEMISTRY, (1996 FEB 16) 271 (7) 3917-24, XP002094746
- KIM E ET AL: "DNA-conformation is an important determinant of sequence-specific DNA binding by tumor suppressor p53 [published erratum appears in Oncogene 1997 Nov 6;15(19):2385]." ONCOGENE, (1997 AUG 14) 15 (7) 857-69, XP002094747

## Beschreibung

Die vorliegende Erfindung betrifft ein Verfahren zur Beeinflussung der Bindung von p53 an Zielgene. p53 ist ein in Tier- und Menschenzellen vorliegendes Protein, das als "Wächter des Genoms" bezeichnet wird. p53 ist ein sequenzspezifischer Transaktivator, der bei DNA-Schäden aktiviert wird. In dieser Form bindet p53 an Promotoren von Zielgenen und aktiviert deren Transkription. Dadurch wird ein Wachstumsstillstand der Zellen mit anschließender Reparatur der DNA-Schäden bzw. der Tod der Zellen erreicht.

Es hat sich gezeigt, daß p53 in vielen Tumoren sein Transaktivator-Aktivität verloren hat. Dies liegt oftmals daran, daß die Bindung von p53 an die Promotoren von Zielgenen gestört ist oder nicht die gewünschten Zielgene reguliert werden.

Der vorliegenden Erfindung liegt somit die Aufgabe zugrunde, ein Mittel bereit zu stellen, mit dem die Bindung von p53 an Zielgene beeinflußt werden kann.

Erfindungsgemäß wird dies durch die Gegenstände in den Patentansprüchen erreicht.

Gegenstand der vorliegenden Erfindung ist somit ein Verfahren zur Beeinflussung der Bindung von p53 an ein Zielgen, bei dem die Konformationen von p53 und des Zielgens, insbesondere mittels Konformationsmodulatoren; aufeinander abgestimmt werden und die Bindung von p53 direkt oder indirekt nachgewiesen wird.

Die vorliegende Erfindung beruht auf der Erkenntnis des Anmelders, daß p53-Bindungssequenzen in den Promotoren von Zielgenen nicht nur in der "normalen" B-Duplex-DNA-Konformation sondern auch in non-B-DNA-Konformationen (z.B. Kruziforme) vorliegen können. Ferner hat der Anmelder erkannt, daß p53 seine Bindungssequenzen sowohl in der B-Duplex-DNA-Konformation als auch in non-B-DNA-Konformationen erkennt. Die Erkennung ist allerdings unterschiedlich, abhängig von der Konformation von p53.

Erfindungsgemäß werden diese Erkenntnisse dazu genutzt, die Bindung von p53 an Zielgene zu beeinflussen, indem die Konformationen von p53 und der Zielgene, insbesondere mittels Konformationsmodulatoren, aufeinander abgestimmt werden und die Bindung von p53 direkt oder indirekt nachgewiesen wird.

Der Ausdruck "p53" umfaßt ein p53 jeglicher Art und Abstammung. Dieses kann eine Wildtyp-Sequenz aufweisen oder ein mutiertes p53 sein. Letztes ist vorzugsweise ein solches p53, bei dem die Fähigkeit zur DNA-Bindung mutiert ist. Auch kann p 53 in Form eines Fragmentes von p53 vorliegen, das für die DNA-Bindung verantwortlich ist. Desweiteren kann p53 in Form eines Fusionspolypeptids vorliegen. Dieses kann auch, wie jedes andere p53, in Form eines es kodierenden Vektors vorliegen.

Der Ausdruck "Zielgene" umfaßt Gene jeglicher Art und Abstammung, deren Expression durch p53 reguliert werden. Beispiele solcher Gene sind RGC, MCK, mdm2, Cyclin G, synthetische p53-Reportergene, p21 und bax, wobei p21 und insbesondere bax bevorzugt sind. p21 wird für den durch p53 bedingten Wachstumsstillstand der Zelle und bax für den durch p53 bedingten Zelltod verantwortlich gemacht. Insbesondere umfaßt der Ausdruck "Zielgene" die Promotorsequenzen dieser und ganz besonders p53-Bindungssequenzen davon. Die Zielgene können in jeglicher DNA-Konformation vorliegen. Sie können in Zellen, insbesondere Tumorzellen, oder isoliert vorliegen. Auch können die Zielgene in Verbindung mit weiteren Sequenzen, insbesondere mit solchen, die für ein Reporterprotein kodieren, vorliegen.

Der Ausdruck "Konformationsmodulatoren" umfaßt Stoffe jeglicher Art, die einen Konformationswechsel einer Nukleinsäure hervorrufen können. Insbesondere handelt es sich um Stoffe, die eine DNA von der B-Duplex-DNA-Konformation in eine non-B-DNA-Konformation oder umgekehrt umwandeln können. Beispiele solcher Stoffe sind interkalierende Stoffe. Ferner umfaßt der Ausdruck "Konformationsmodulatoren" auch Stoffe jeglicher Art, die eine Konformationsänderung von p53 bewirken können. Beispiele solcher Stoffe sind jene, welche die carboxyterminale Regulatorregion von p53, z.B. der Antikörper pAB 421, modifizieren können.

Der Ausdruck "direkter oder indirekter Nachweis der Bindung von p53" umfaßt jeglichen Nachweis für eine solche Bindung. Beispiele eines direkten Nachweises umfassen Verfahren mit denen die Bindung.von p53 an DNA gezeigt werden kann. Indirekte Nachweise umfassen Verfahren mit denen die Folgen einer p53-DNA-Bindung, z.B. die Regulation der Expression von Reportergenen bzw. biologische Folgeerscheinungen, wie Wachstumsstillstand von Zellen oder deren Tod, gezeigt werden können.

Ein erfindungsgemäßes Verfahren kann in üblicher Weise durchgeführt werden. Beispielsweise können p53-Bindungssequenzen in der B-Duplex DNA-Konformation bzw. in einer non-B-DNA-Konformation mit p53 und gegebenenfalls Konformatiovsmodulatoren inkubiert werden und die DNA-Bindung von p53 wird direkt nachgewiesen. Auch können Zellen, insbesondere Tumorzellen, mit gestörter DNA-Bindung von p53 mit Konformationsmodulatoren inkubiert werden und nach Bestrahlung der Zellen wird die DNA-Bindung.von p53 indirekt über den Wachstumsstillstand bzw. den Tod der Zellen nachgewiesen.

Mit der vorliegenden Erfindung ist es möglich, die Bindung von p53 an Zielgene zu beeinflussen. Dies kann genutzt werden, um eine gestörte DNA-Bindung von p53 zu korrigieren. Eine solche liegt häufig bei Tumorzellen vor. Weiterhin kann die DNA-Bindungsspezifität von p53 so beeinflußt werden, daß bestimmte, gewünschte Zielgene reguliert werden. Somit eignet sich die vorliegende Erfindung als zumindest begleitende Therapiemaßnahme bei der Bekämpfung von Tumorerkrankungen.

Ferner bietet die vorliegende Erfindung die Möglichkeit, Stoffe zu finden, die sich als Konformationsmodulatoren von p53 und/oder seinen Zielgenen eignen können. Hierzu wird das erfindungsgemäße Verfahren dahingehend durchgeführt, daß anstelle bekannter Konformationsmodulatoren unbekannte eingesetzt und die bekannten Konformationsmodulatoren gegebenenfalls als Kontrollen verwendet werden.

### Kurze Beschreibung der Zeichnungen:

- Fig. 1: zeigt die DNA-Konformationen von Oligonukleotiden, die p53-Bindungssequenzen von RGC enthalten,
- Fig. 2: zeigt die DNA-Bindung von p53 an Oligonukleotide mit p53-Bindungssequenzen von RGC, wobei die Oligonukleotide in verschiedenen DNA-Konformationen vorliegen. Die Spuren 1 und 2 stellen Kontrollen dar,
- Fig. 3: zeigt die DNA-Konformationen von Oligonukleotiden, die p53-Bindungssequenzen der von Hupp gefundenen Sequenz enthalten,
- Fig. 4: zeigt die DNA-Bindung von p53 an Oligonukleotide mit p53-Bindungssequenzen der Hupp-Sequenz, wobei die Oligonukleotide in verschiedenen DNA-Konformationen vorliegen.

Die Erfindung wird durch das nachstehende Beispiel erläutert.

### Beispiel: Bindung von p53 an ein Zielgen

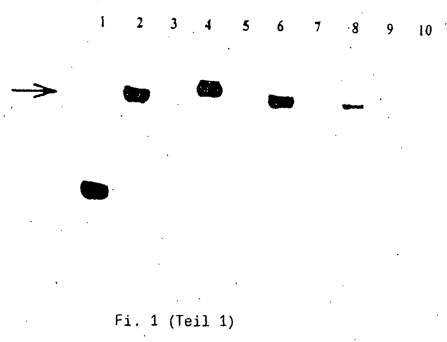

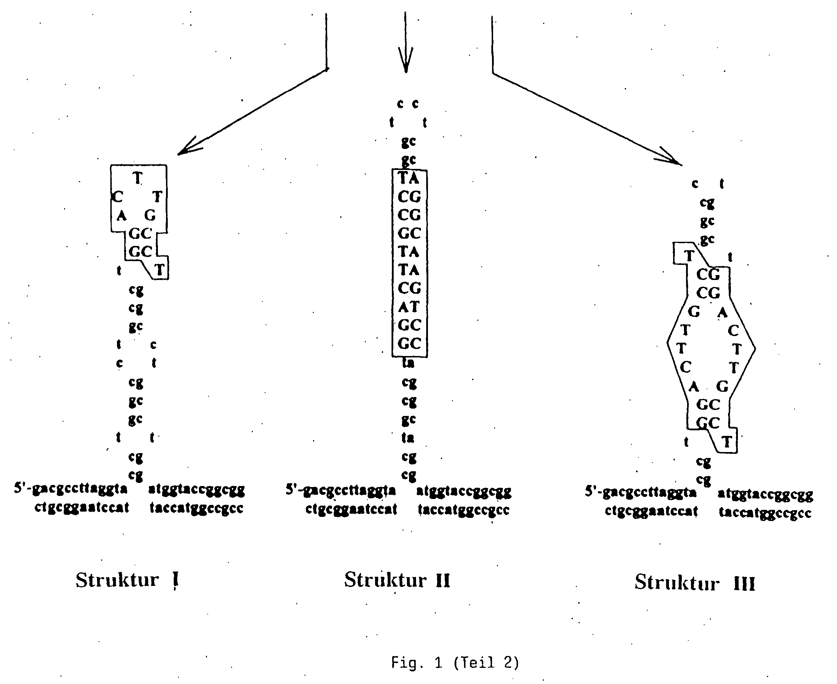

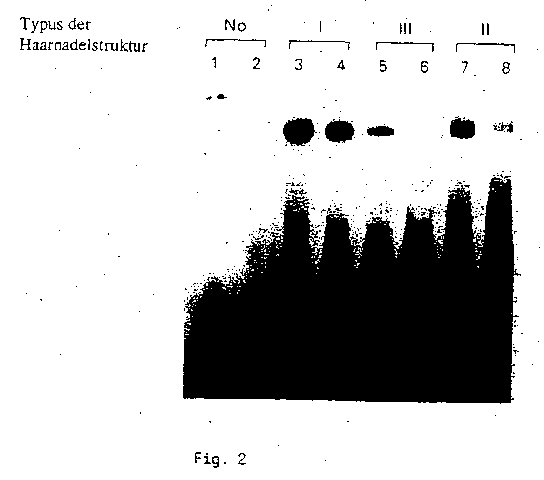

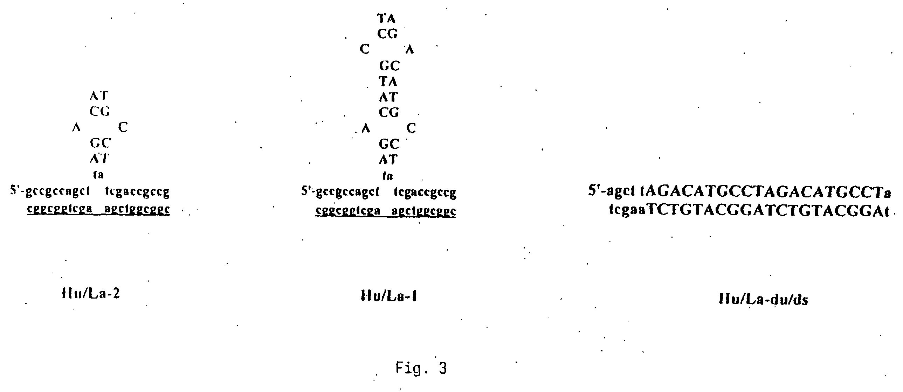

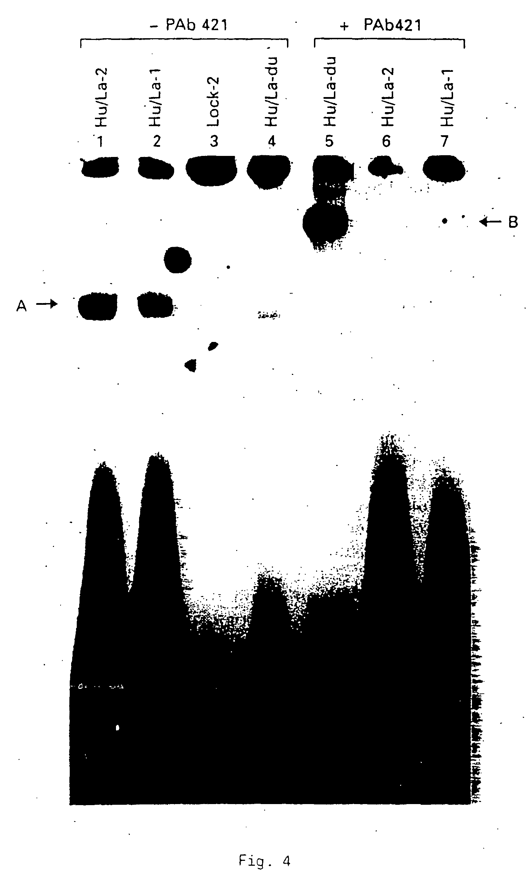

**(a) Bindung von p53 an p53-Bindungssequenzen von RGC**
   Aus Sf9-lnsektenzellen, die mit rekombinanten, Wildtyp p53 exprimierenden Baculoviren infiziert waren, wurde p53 in üblicher Weise isoliert. p53 hatte eine Reinheit von mehr als 80 % in einem SDS-Polyacrylamidgel. p53 wurde in eine Bindungsreaktion mit Oligonukleotiden eingesetzt. Zur Herstellung dieser Oligonukleotide wurde von den nachstehenden Oligonukleotiden ausgegangen: RGC-1b/ss 5'-gacgccttaggtagggccctGGACTTGCCTcccgggatggtaccgcgg-3' RGC-1a/ss 5'-gacgccttaggtacctggcctgcctGGACTTGCCTggcctgcctggatggtaccgcgg-3' 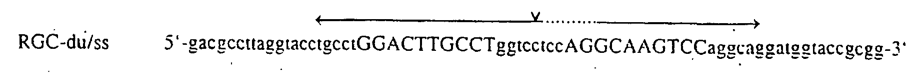

RGC-du/ss 5'-gacgccttaggtacctgcctGGACTTGCCTggtcctccAGGCAAGTCCaggcaggatggtaccgcgg-3'
   Diese Oligonukleotide enthalten 1-2 "Halfsites" der p53-Bindungssequenzen von RGC und jeweils identische terminale Sequenzen an ihren 5'- und 3'-Enden. Die Oligonukleotide wurden in eine "Annealing"-Reaktion mit dem nachstehenden Oligonukleotid eingesetzt, das komplementäre Sequenzen zu den terminalen Sequenzen aufweist: Lock 5'-ccgcggtaccattacctaaggcgtc-3'
   Es wurden Oligonukleotide erhalten, die in der Duplex-B-DNA-Konformation (Struktur II) bzw. in non-B-DNA-Konformationen (Strukturen I bzw. III) vorliegen (vgl. Fig. 1). Diese Oligonukleotide wurden radioaktiv endmarkiert und in die Bindungsreaktion mit p53 eingesetzt. Die Reaktion erfolgte während 30 Min. bei Raumtemperatur. Die Reaktionsprodukte wurden einer Polyacrylamid-Gelelektrophorese unterzogen.
   Es zeigte sich, daß p53 an die Oligonukleotide der drei DNA-Konformationen bindet, jedoch unterschiedlich stark. Die stärkste Bindung findet sich bei einer non-B-DNA-Konformation (Struktur I), während die Bindung an die Duplex-B-DNA=Konformation (Struktur II) bzw. an eine weitere non-B-DNA-Konformation (Struktur III) schwächer ist (vgl. Fig. 2). Somit wird deutlich, daß die Bindung von p53 an ein Zielgen durch Konformationsänderung des Zielgens beeinflußt werden kann.
**(b) Bindung von p53 an p53-Bindungssequenzen der Hupp-Sequenz**
   Entsprechend der Beschreibung von (a) wurde p53 in eine Bindungsreaktion mit Oligonukleotiden eingesetzt. Zu deren Herstellung wurde von den nachstehenden Oligonukleotiden ausgegangen: 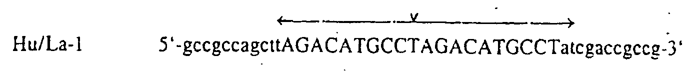
Hu/La-1 5'-gccgccagcttAGACATGCCTAGACATGCCTatcgaccgccg-3'
 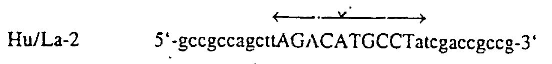
Hu/La-2    5'-gccgccagcttAGACATGCCTatcgaccgccg-3'
 Hu/La-du 5'-agcttAGACATGCCTAGACATGCCTa-3'
   Diese enthalten 1-2 "Halfsites" der von Hupp gefundenen p53-Bindungssequenzen. Diese p53-Bindungssequenzen zeichnen sich dadurch aus, daß p53 erst nach Aktivierung mit dem Antikörper PAb 421 in der Lage ist, an sie zu binden. Die Oligonukleotide wurden in eine "Annealing" Reaktion mit dem in (a) angegebenen Oligonukleotid "Lock" eingesetzt. Es wurden Oligonukleotide erhalten, die in der Duplex-B-DNA-Konformation (Hu/La-du/ds), bzw. in non-B-DNA-Konformationen (Hu/La-2 bzw. Hu/La-1) vor (vgl. Fig. 3) vorliegen.
   Es zeigte sich, daß p53 an die Oligonukleotide der drei DNA-Konformationen bindet, jedoch unterschiedlich. An das Oligonukleotid in der Duplex-B-DNA-Konformation bindet p53 erst nach Aktivierung von p53 durch den Antikörper PAb421, während seine Bindung an non-B-DNA-Konformationen ohne Modifizierung von p53 erfolgt (vgl. Fig. 4). Andererseits bewirkt die Bindung von PAb 421 an p53 eine Inhibition der p53-Bindung an dieses Oligonukleotid in der non-B-DNA-Konformation. Somit wird deutlich, daß die Bindung von p53 an ein Zielgen durch Konformationsänderungen des Zielgens und von p53 beeinflußt werden kann.

## Patentansprüche

1. In vitro Verfahren zur Beeinflussung der Bindung von p53 an ein Zielgen, bei dem die Konformationen von p53 und des Zielgens aufeinander abgestimmt werden und die Bindung von p53 direkt oder indirekt nachgewiesen wird, wobei zumindest die Konformation des Zielgens **dadurch** beeinflußt wird, daß ein Konformationsmodulator einen Konformationswechsel der p53-Bindungssequenzen im Promotor des Zielgens von der B-Duplex-DNA-Konformation in eine non-B-DNA-Konformation oder umgekehrt durchführt.

2. Verfahren nach Anspruch 1, wobei ferner die Konformation von p53 beeinflußt wird.

3. Verfahren nach Anspruch 1 oder 2, wobei die Abstimmung der Konformationen über einen oder mehrere Konformationsmodulatoren erfolgt.

4. Verfahren nach einem der Ansprüch 1 bis 3, wobei p53 ein mutiertes p53 ist.

5. Verfahren nach Anspruch 4, wobei die Mutation von p53 die Fähigkeit zur DNA-Bindung betrifft.

6. Verfahren nach einem der Ansprüche 1 bis 5, wobei das Zielgen p21 oder bax ist.

7. Verfahren nach einem der Ansprüche 1 bis 6, wobei der Konformationsmodulator des Zielgens ein interkalierender Stoff ist.

8. Verfahren nach einem der Ansprüche 2 bis 7, wobei der Konformationsmodulator von p53 die carboxyterminale Regulatorregion von p53 betrifft.

9. Verfahren nach Anspruch 8, wobei der Konformationsmodulator der Antikörper PAb421 ist.

## Claims

1. An in vitro method for influencing the p53 binding to a target gene, wherein the conformations of p53 and target gene are coordinated and the p53 binding is detected directly or indirectly, wherein at least the conformation of the target gene is influenced by a conformation modulator carrying out a conformation change of the p53 binding sequences in the target gene promoter of the B-duplex DNA conformation into a non-B-DNA conformation or *vice versa.*

2. The method according to claim 1, wherein the conformation of p53 is also influenced.

3. The method according to claim 1 or 2, wherein the conformation is coordinated via one or several conformation modulators.

4. The method according to claim 1 to 3, wherein p53 is a mutated p53.

5. The method according to claim 4, wherein the mutation of p53 relates to the ability of DNA binding.

6. The method according to any one of claims 1 to 5, wherein the target gene is p21 or bax.

7. The method according to any one of claims 1 to 6, wherein the conformation modulator of the target gene is an intercalating substance.

8. The method according to any one of claims 2 to 7, wherein the conformation modulator of p53 relates to the carboxy-terminal regulator region of p53.

9. The method according to claim 8, wherein the conformation modulator is the PAb421 antibody.

## Revendications

1. Procédé *in vitro* pour influencer la liaison de p53 à un gène cible, dans lequel les conformations du p53 et du gène cible sont réglées l'une sur l'autre, et la liaison du p53 apparaît être directe ou indirecte, dans lequel au moins la conformation du gène cible est influencée par le fait qu'un modulateur de conformation d'un échange de conformation des séquences de liaison du p53 dans le promoteur du gène cible de la conformation B-Duplex-ADN est réalisé par une conformation non-B-ADN ou inversement.

2. Le procédé selon la revendication 1, dans lequel en outre la conformation de p53 est influencée.

3. Un procédé selon la revendication 1 ou 2, dans lequel la détermination de la conformation est réalisée par un ou plusieurs modulateurs de conformation.

4. Un procédé selon l'une quelconque des revendications 1 à 3, dans lequel le p53 est un p53 muté.

5. Le procédé selon la revendication 4, dans lequel la mutation de p53 satisfait aux nécessités de la liaison ADN.

6. Un procédé selon l'une quelconque des revendications 1 à 5, dans lequel le gène cible est p21 ou bax.

7. Un procédé selon l'une quelconque des revendications 1 à 6, dans lequel le modulateur de conformation du gène cible est une substance d'intercalation.

8. Un procédé selon l'une quelconque des revendications 2 à 7, dans lequel le modulateur de conformation de p53 correspond à la région de régulation carboxyterminale de p53.

9. Le procédé selon la revendication 8, dans lequel le modulateur de conformation est un anticorps PAb421.
